# EUROPÄISCHE PATENTSCHRIFT

(11) **EP 1 443 890 B1**
(45) Veröffentlichungstag und Bekanntmachung des Hinweises auf die Patenterteilung: **13.01.2010**
(21) Anmeldenummer: 02787631.7
(22) Anmeldetag: 11.11.2002
(51) Int. Cl.: A61K 8/41, A61K 8/49, A61Q 5/10

(54) **OXIDATIONSFÄRBEMITTEL AUF DER BASIS ZWEIKERNIGER ENTWICKLERKOMPONENTEN**
OXIDATION COLOURING AGENT BASED ON BICYCLIC DEVELOPER COMPONENTS
COLORANTS D'OXYDATION A BASE DE REVELATEURS BICYCLIQUES

(30) Priorität: 17.11.2001 DE 10156667; 20.11.2001 DE 10156881
(43) Veröffentlichungstag der Anmeldung: 11.08.2004
(73) Patentinhaber: Henkel AG & Co. KGaA, 40589 Düsseldorf (DE)
(72) Erfinder: ROSE, David, 40723 Hilden (DE); HÖFFKES, Horst, 40595 Düsseldorf (DE); MEINIGKE, Bernd, 51381 Leverkusen (DE); KLEEN, Astrid, 40699 Erkrath (DE)
(86) Internationale Anmeldenummer: PCT/EP2002/012557
(87) Internationale Veröffentlichungsnummer: WO 2003/041670

(56) Entgegenhaltungen:
- WO-A-00/61091
- WO-A-01/41716
- WO-A-01/97766
- WO-A-97/31886

## Beschreibung

Die vorliegende Erfindung betrifft Mittel zum Färben keratinischer Fasern, die ein zweikerniges p-Aminophenolderivat als Entwicklerkomponente in Kombination mit mindestens einer weiteren speziellen Entwicklerkomponente enthalten, ein Verfahren zum Färben keratinischer Fasern mit diesen Mitteln sowie die Verwendung dieser Farbstoffkombination zur Färbung keratinischer Fasern.

Keratinfasern, insbesondere das menschliche Haar werden heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so dass eine vielfach unerwünschte Nuancenverschiebung oder gar eine sichtbare "Entfärbung" eintreten kann.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Schweiß, Reibung und den Einfluss chemischer Reduktionsmittel, z. B. Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie dennoch nicht den individuellen Wünschen der einzelnen Person entspricht und rückgängig gemacht werden soll.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkomponenten, u. a. für die Erzielung von Färbungen im Rot- und Braun-Bereich.

Es wurde nunmehr überraschenderweise gefunden, dass besonders intensive Färbungen mit ausgezeichneten Echtheitseigenschaften und außerordentlich guten toxikologischen Eigenschaften erhalten werden können, wenn Färbemittel eingesetzt werden, die zweikernige Entwicklerkomponenten auf der Basis von p-Aminophenolen in Kombination mit speziellen Entwicklerkomponenten, ausgewählt aus der Gruppe der Heterocyclen, die gebildet wird aus Pyrimidinderivaten, Pyrazolderivaten sowie Pyrazol-Pyrimidinderivaten, enthalten.

Ein erster Gegenstand der vorliegenden Erfindung sind daher Mittel zum Färben keratinischer Fasern, insbesondere von menschlichen Haaren, enthaltend in einem zum Färben geeigneten Medium als Oxidationsfarbstoffvorprodukte eine Kombination aus Bis-(5-amino-2-hydroxyphenyl)-methan oder einem seiner physiologisch verträglichen Salze als Entwicklerkomponente und
b) mindestens einer weiteren, heterocyclischen Entwicklerkomponente, ausgewählt aus der Gruppe der
   - Pyrimidinderivate,
   - Pyrazolderivate und der
   - Pyrazol-Pyrimidin-Derivate,
   sowie deren physiologisch verträglichen Salzen.

Unter keratinischen Fasern werden erfindungsgemäß Pelze, Wolle, Federn und insbesondere menschliche Haare verstanden.

In der WO 97/31886 werden oxidative Färbemittel beschrieben, die Bis-(5-amino-2-hydroxyphenyll)-methan enthalten.

Bevorzugte Pyrimidinderivate sind erfindungsgemäß solche, die aus Verbindungen gemäß der allgemeinen Formel (II) ausgewählt sind, 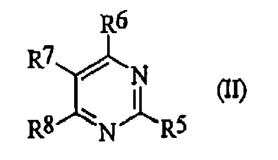 in der
- R⁵ steht für eine Hydroxygruppe oder eine Gruppe -NRR', in der R und R' unabhängig voneinander stehen für Wasserstoff, eine Hydroxyalkyl- oder Alkylgruppe, jeweils mit 1 bis 4 Kohlenstoffatomen, oder sie bilden mit dem sie tragenden Stickstoffatom ein heterocyclisches Ringsystem,
- R⁶, R⁷ und R⁸ stehen unabhängig voneinander für eine Hydroxygruppe, eine Aminogruppe oder eine C₁-C₄-Dialkylaminogruppe,
sowie deren physiologisch verträgliche Salze.

Beispiele für die in den erfindungsgemäßen Verbindungen der allgemeinen Formel (II) genannten C₁-C₄-Dialkylaminosubstituenten sind Dimethyl- und Diethylaminogruppen.

Als besonders bevorzugte Pyrimidinderivate gemäß der allgemeinen Formel (II) haben sich 4-Hydroxy-2,5,6-triaminopyrimidin, 5-Hydroxy-2,4,6-triaminopyrimidin, 2-Dimethyl-amino-4,5,6-triaminopyrimidin und 2,4,5,6-Tetraaminopyrimidin sowie deren physiologisch verträglichen Salze erwiesen.

Bevorzugte Pyrazolderivate sind erfindungsgemäß solche, die aus Verbindungen gemäß der allgemeinen Formel (III) ausgewählt sind, 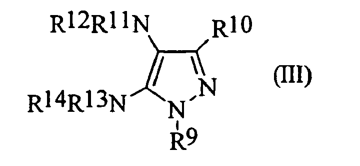 in der
- R⁹, R¹¹, R¹², R¹³ und R¹⁴ unabhängig voneinander stehen für Wasserstoff, eine geradkettige oder verzweigte eine C₁-C₆-Alkylgruppe, eine C₁-C₄-Mono- oder eine C₂-C₄-Polyhydroxyalkylgruppe, eine C₂-C₄-Aminoalkylgruppe, eine Phenylgruppe, die gegebenenfalls mit einer Nitrogruppe, einer Trifluormethylgruppe, oder einer Aminogruppe substituiert ist, eine Benzylgruppe, die gegebenenfalls mit einem Halogenatom, einer C₁-C₄-Alkylgruppe, einer Methylendioxygruppe oder einer Aminogruppe substituiert ist, eine Pyridylgruppe, eine Thienylgruppe, eine Furylgruppe oder eine Gruppe 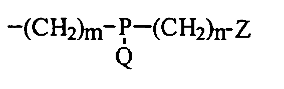 in der m und n unabhängig voneinander ganze Zahlen im Bereich von 1 bis 3 darstellen, P steht für Sauerstoff oder eine Gruppe NH-, Q steht für Wasserstoff oder eine Methylgruppe und Z steht für eine Methylgruppe, eine Gruppe -OR oder -NRR', wobei R und R' unabhängig voneinander Wasserstoff, eine Methyl- oder eine Ethylgruppe sein können und für den Fall, dass R¹³ Wasserstoff ist, R¹⁴ auch C₁-C₄-Alkylamino bedeuten kann und
- R¹⁰ steht für Wasserstoff, eine geradkettige oder verzweigte C₁-C₆-Alkylgruppe, eine C₂-C₄-Hydroxyalkylgruppe, eine C₁-C₄-Aminoalkylgruppe, eine N-C₁-C₄-Alkylamino-C₁-C₄-alkylgruppe, eine C₁-C₄-Alkoxymethylgruppe, eine Phenylgruppe, die gegebenenfalls mit einem Halogenatom, einer C₁-C₄-Alkylgruppe, einer C₁-C₄-Alkoxygruppe, einer Aminogruppe, einer Trifluormethylgruppe, einer C₁-C₄-Aminoalkylgruppe oder einer Nitrogruppe substituiert ist, einen Heterocyclus, der unter Thiophen, Furan und Pyridin ausgewählt ist, oder eine Gruppe -(CH₂)ₚ-O-(CH₂)_{q}-OR', worin R' für Wasserstoff oder eine Methylgruppe steht und p und q ganze Zahlen sind, die unabhängig voneinander im Bereich von 1 bis 3 liegen, oder für Wasserstoff mit der Maßgabe, dass dann R₉ von einer unsubstituierten oder substituierten Benzylgruppe verschieden ist.

Als Beispiele für die in den erfindungsgemäßen Verbindungen der allgemeinen Formel (III) genannten Substituenten gelten die in der unter den allgemeinen Formeln (I) und (II) bereits genannten. Weitere Beispiele für die als Substituenten in den erfindungsgemäßen Verbindungen der Formel (III) genannten C₁- bis C₆-Alkylgruppen sind die Gruppen Methyl, Ethyl, Propyl, Isopropyl und Butyl, Pentyl und Hexyl. Ethyl und Methyl sind bevorzugte Alkylgruppen. Erfindungsgemäß bevorzugte C₁- bis C₄-Alkoxygruppen sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispielhafte erfindungsgemäße C₂-C₄-Aminoalkylgruppen sind 2-Aminoxyethyl-, eine 3-Aminopropyl- oder eine 4-Aminobutylgruppe. Die weiteren verwendeten Begriffe leiten sich erfindungsgemäß von den hier gegebenen Definitionen ab.

Als besonders bevorzugte Pyrazolderivate gemäß der allgemeinen Formel (III) haben sich 4,5-Diaminopyrazol, 4,5-Diamino-1-(2`-hydroxyethyl)-pyrazol, 4,5-Diamino-1,3-dimethyl-pyrazol, 4,5-Diamino-3-methyl-1-phenyl-pyrazol, 4,5-Diamino-1-methyl-3-phenyl-pyrazol, 1-Benzyl-4,5-Diamino-3-methyl-pyrazol, 4,5-Diamino-1-tert.-butyl-3-methyl-pyrazol, 4,5-Diamino-1-(2'-hydroxyethyl)-3-methyl-pyrazol, 4,5-Diamino-1-ethyl-3-methyl-pyrazol, 4,5-Diamino-1-ethyl-3-hydroxymethyl-pyrazol, 4,5-Diamino-3-hydroxymethyl-1-methyl-pyrazol, 4,5-Diamino-3-hydroxymethyl-1-isopropyl-pyrazol oder eines der physiologisch verträglichen Salze dieser Verbindungen erwiesen.

Ein ganz besonders bevorzugtes Pyrazolderivat gemäß der allgemeinen Formel (III) ist das 4,5-Diamino-1-(2'-hydroxyethyl)-pyrazol.

Bevorzugte Pyrazol-Pyrimidin-Derivate sind erfindungsgemäß solche, die aus Verbindungen gemäß der allgemeinen Formel (IV) ausgewählt sind, 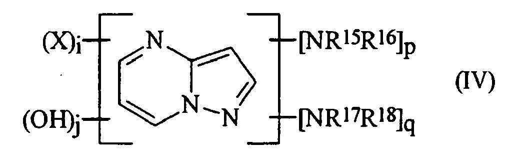 wobei:
- R¹⁵, R¹⁶, R¹⁷ und R¹⁸ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die Reste X stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- j hat den Wert 0 oder 1,
   mit der Maßgabe, dass
- die Summe aus p + q ungleich 0 ist,
- wenn p + q gleich 2 ist, n den Wert 0 hat, und die Gruppen NR¹⁵R¹⁶ und NR¹⁷R¹⁸ belegen die Positionen (2,3); (5,6); (6,7); (3,5) oder (3,7);
- wenn p + q gleich 1 ist, n den Wert 1 hat, und die Gruppen NR¹⁵R¹⁶ (oder NR¹⁷R¹⁸) und die Gruppe OH belegen die Positionen (2,3); (5,6); (6,7); (3,5) oder (3,7).

Als Beispiele für die in den erfindungsgemäßen Verbindungen der allgemeinen Formel (IV) genannten Substituenten gelten die in den unter den allgemeinen Formeln (I) bis (III) bereits genannten. Beispiele für erfindungsgemäße Di-(C₁- bis C₄-hydroxyalkyl)-aminoreste sind Di-(2-hydroxyethyl)-amino-, Di-(3-hydroxypropyl)-amino- und Di-(4-hydroxybutyl)-aminogruppen.

Als besonders bevorzugte Pyrazol-Pyrimidin-Derivate gemäß der allgemeinen Formel (IV) haben sich Pyrazol-[1,5-a]-pyrimidin-3,7-diamin, 2,5-Dimethyl-pyrazol-[1,5-a]-pyrimidin-3,7-diamin, Pyrazol-[1,5-a]-pyrimidin-3,5-diamin, 2,7-Dimethyl-pyrazol-[1,5-a]-pyrimidin-3,5-diamin, 3-Aminopyrazol-[1,5-a]-pyrimidin-7-ol, 3-Aminopyrazol-[1,5-a]-pyrimidin-5-ol, 2-(3-Aminopyrazol-[ 1,5-a]-pyrimidin-7-ylamino)-ethanol, 2-(7-Aminopyrazol-[1,5-a]-pyrimidin-3-ylamino)-ethanol, 2-[(3-Aminopyrazol-[1,5-a]-pyrimidin-7-yl)-(2-hydroxy-ethyl)-amino]-ethanol, 2-[(7-AminopyrazoI-[l,5-a]-pyrimidin-3-yI)-(2-hydroxy-ethyl)-amino]-ethanol, 5,6-Dimethylpyrazol-[1,5-a]-pyrimidin-3,7-diamin, 2,6-Dimethylpyrazol-[1,5-a]-pyrimidin-3,7-diamin und 3-Amino-7-dimethylamino-2,5-dimethylpyrazol-[1,5-a]-pyrimidin oder eines der physiologisch verträglichen Salze dieser Verbindungen erwiesen.

Die erfindungsgemäßen farbverändernden Mittel können neben den erfindungsgemäßen Farbstoffkombinationen mindestens ein weiteres Farbstoffvorprodukt enthalten.

Hinsichtlich der in den erfindungsgemäßen Mitteln einsetzbaren Farbstoffvorprodukten unterliegt die vorliegende Erfindung keinerlei Einschränkungen. Die erfindungsgemäßen Mittel können als Farbstoffvorprodukte
- Oxidationsfarbstoffvorprodukte vom Entwickler- und Kuppler-Typ, und
- Vorstufen naturanaloger Farbstoffe, wie Indol- und Indolin-Derivate,
sowie Mischungen von Vertretern dieser Gruppen enthalten sein.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate und heterocyclische Hydrazone eingesetzt. Es kann erfindungsgemäß bevorzugt sein, als weitere Entwicklerkomponente ein p-Phenylendiaminderivat oder eines seiner physiologisch verträglichen Salze einzusetzen. Besonders bevorzugt sind p-Phenylendiaminderivate der Formel (V) 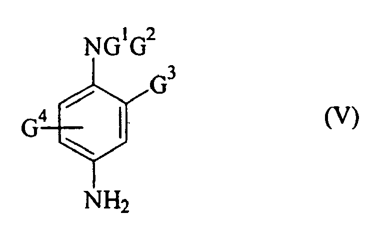 wobei
- G¹ steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein 4'-Aminophenylradikal oder ein C₁- bis C₄-Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal oder ein C₁ - bis C₄-Alkylradikal, das mit einer stickstoffhaltigen Gruppe substituiert ist;
- G³ steht für ein Wasserstoffatom, ein Halogenatom, wie ein Chlor-, Brom, Jod- oder Fluoratom, ein C₁- bis C₄-Alkylradikal, ein C₁- bis C₄-Monohydroxyalkylradikal, ein C₁- bis C₄-Hydroxyalkoxyradikal, ein C₁- bis C₄-Acetylaminoalkoxyradikal, ein C₁- bis C₄- Mesylaminoalkoxyradikal oder ein C₁- bis C₄-Carbamoylaminoalkoxyradikal;
- G⁴ steht für ein Wasserstoffatom, ein Halogenatom oder ein C₁- bis C₄-Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkyläminogruppen, Imidazolinium und Ammonium.

Besonders bevorzugte p-Phenylendiamine der Formel (V) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylamino-ethyloxy)-p-phenylendiamin, N-(β-Methoxyethyl)-p-phenylendiamin und 5,8-Diamino-benzo-1,4-dioxan sowie ihren physiologisch verträglichen Salzen.

Erfindungsgemäß ganz besonders bevorzugte p-Phenylendiaminderivate der Formel (V) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (VI) entsprechen, sowie ihre physiologisch verträglichen Salze: 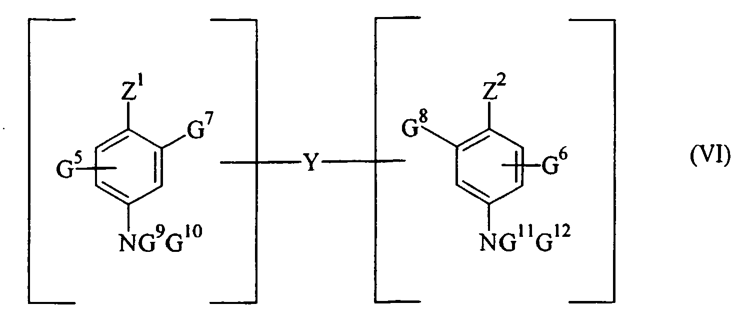 wobei:
- Z¹ und Z² stehen unabhängig voneinander für ein Hydroxyl- oder NH₂-Radikal, das gegebenenfalls durch ein C₁- bis C₄-Alkylradikal, durch ein C₁- bis C₄-Hydroxyalkylradikal und/oder durch eine Verbrückung Y substituiert ist oder gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder C₁- bis C₈-Alkoxyradikale substituiert sein kann, oder eine direkte Bindung,
- G⁵ und G⁶ stehen unabhängig voneinander für ein Wasserstoff- oder Halogenatom, ein C₁- bis C₄-Alkylradikal, ein C₁- bis C₄-Monohydroxyalkylradikal, ein C₂- bis C₄- Polyhydroxyalkylradikal, ein C₁- bis C₄-Aminoalkylradikal oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder ein C₁- bis C₄-Alkylradikal,
mit der Maßgabe, dass die Verbindungen der Formel (VI) nur eine Verbrückung Y pro Molekül enthalten.

Die in Formel (VI) verwendeten Substituenten sind erfindungsgemäß analog zu den obigen Ausführungen definiert.

Bevorzugte zweikernige Entwicklerkomponenten der Formel (VI) sind insbesondere: N,N'-bis-(β-Hydroxyethyl)-N,N'-bis-(4'-aminophenyl)-1,3-diamino-propan-2-ol, N,N'-bis-(β-Hydroxyethyl)-N,N'-bis-(4'-aminophenyl)-ethylendiamin, N,N'-bis-(4-Aminophenyl)-tetra-methylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N'-bis-(Ethyl)-N,N'-bis-(4'-amino,3'-methylphenyl)-ethylendiamin, 1,8-bis-(2',5'-Diaminophenoxy)-3,5-dioxaoktan, 1,4-Bis-(4'-aminophenyl)-diaza-cycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (VI) sind N,N'-bis-(β-Hydroxyethyl)-N,N'-bis-(4'-aminophenyl)-1,3-diamino-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (VII) 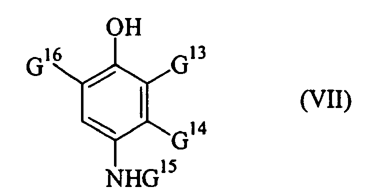 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁ bis C₄-Hydroxyalkyl-(C₁-bis C₄)-aminoalkylrest oder einen (Di-C₁ bis C₄-Alkylamino)-(C₁- bis C₄)-alkylrest, und
- G¹⁴ steht für ein Wasserstoff- oder Halogenatom, einen C₁ bis C₄-Alkylrest, einen C₁- bis C₄-Monohydroxyalkylrest, einen C₂- bis C₄-Polyhydroxyalkylrest, einen (C₁- bis C₄)-Alkoxy-(C₁ bis C₄)-alkylrest, einen C₁- bis C₄-Aminoalkylrest oder einen C₁- bis C₄-Cyanoalkylrest,
- G¹⁵ steht für Wasserstoff, einen C₁ bis C₄-Alkylrest, einen C₁ bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (VII) verwendeten Substituenten sind erfindungsgemäß analog zu den obigen Ausführungen definiert.

Bevorzugte p-Aminophenole der Formel (VII) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (VII) sind p-Aminophenol, 4-Amino-3-methylphenol, 4-Amino-2-aminomethylphenol, 4-Amino-2-(α,β-dihydroxy-ethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-4-chlorphenol oder 2-Amino-5-methylphenol.

Weiterhin kann die Entwicklerkomponente eine weitere heterocyclische Verbindung, wie beispielsweise ein Pyridinderivat oder eines seiner physiologisch verträglichen Salze sein.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Erfindungsgemäß ganz besonders bevorzugte weitere Entwicklerkomponenten sind ausgewählt aus p-Phenylendiamin, p-Toluylendiamin, p-Aminophenol, 1-(2'-Hydroxyethyl)-2,5-diaminobenzol, 1, 10-Bis-(2',5'-diaminophenyl)-1,4,7,10-tetra-oxydecan, N,N-Bis-(2'-hydroxyethyl)-p-phenylendiamin, 4-Amino-3-methylphenol, 4-Amino-2-((diethylamino)-methyl)-phenol, 4-Amino-2,6-dichlorphenol oder 2-Aminomethyl-4-aminophenol sowie den physiologisch verträglichen Salzen dieser Verbindungen. Es kann erfindungsgemäß bevorzugt sein, dem Farbstoffgemisch weiterhin noch eine Kupplerkomponente hinzuzufügen.

Als Kupplerkomponenten werden in der Regel m-Phenylendiaminderivate, Naphthole, Resorcin und Resorcinderivate, Pyrazolone, Pyridinderivate und m-Aminophenolderivate verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxy-ethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte Kupplerkomponenten sind 1-Naphthol, 1,5-Dihydroxynaphthalin, 1,7-Dihydroxynaohthalin, 2,7-Dihydroxynaphthalin, 3-Aminophenol, 5-Amino-2-methylphenol, 5-(2'-Hydroxyethylamino)-3-methylphenol, 2,4-Dichlor-3-aminophenol, 2-Chlor-6-methyl-3-aminophenol, 2-Methyl-4-chlor-5-aminophenol, Resorcin, Resorcinmonomethylester, 2-Chlorresorcin, 4-Chlorresorcin, 2-Methylresorcin, 5-Methylresorcin, 2,5-Dimethylresorcin, m-Phenylendiamin, 2-Methylamino-3-amino-6-methoxypyridin, 1,3-Bis-(2',4'-diaminophenoxy)-propan, 2-Amino-3-hydroxypyridin, 2,4-Diaminophenoxyethanol oder 2,6-Dihydroxy-3,4-dimethylpyridin sowie die physiologisch verträglichen Salzen dieser Verbindungen.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die Entwicklerkomponenten sind in den erfindungsgemäßen Mitteln bevorzugt in Mengen von 0,005 bis 20 Gew.-%, vorzugsweise 0,1 bis 5 Gew.-% und die Kupplerkomponenten sind bevorzugt in Mengen von 0,005 bis 20 Gew.-%, vorzugsweise 0,1 bis 5 Gew.-%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (VIII), 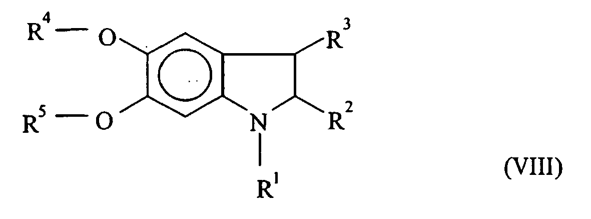 in der unabhängig voneinander
R¹ steht für Wasserstoff, eine C₁-C₄-Alkylgruppe oder eine C₁-C₄-Hydroxy-alkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IX), 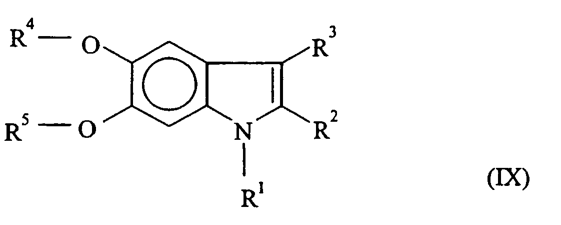 in der unabhängig voneinander
R¹ steht für Wasserstoff, eine C₁-C₄-Alkylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol_{;} N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

Neben den Farbstoffvorprodukten können die erfindungsgemäßen Mittel zur weiteren Nuancierung direktziehende Farbstoffe enthalten. Diese sind üblicherweise ausgewählt aus Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethyl-amino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Die erfindungsgemäßen Mittel enthalten Farbstoffvorprodukte bevorzugt in einem geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wässrig-alkoholischen Lösungen sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Man kann aber die Oxidationsfärbemittel auch zusammen mit einem Katalysator auf das Haar aufbringen, der die Oxidation der Farbstoffvorprodukte, z. B. aktiviert. Solche Katalysatoren sind z. B. Übergangsmetallverbindungen, Iodide, Chinone oder bestimmte Enzyme. Geeignete Enzyme sind z. B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z. B.
- Pyranose-Oxidase und z. B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche Haarfärbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.
Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂₋ bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammonium-chloride und Trialkylmethylammoniumchloride, z. B. Cetyltrimethylammoniumchlorid, Stearyltrimethylammoniumchlorid, Distearyldimethylammoniumchlorid, Lauryldimethyl-ammoniumchlorid, Lauryldimethylbenzylammoniumchlorid und Tricetylmethylammonium-chlorid, sowie die unter den INCI-Bezeichnungen Quaternium-27 und Quatemium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Ein Beispiel für ein als kationisches Tensid einsetzbares quaternäres Zuckerderivat stellt das Handelsprodukt Glucquat^{®} 100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.

Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafqua^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quatemium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der Erfindung ist ein Verfahren zur Färbung von Keratinfasern, dadurch gekennzeichnet, dass es nach einem der Ansprüche 1 bis 23 in einer geeigneten kosmetischen Zubereitung auf die zu färbenden Fasern aufgebracht und nach einer Einwirkungszeit wieder ausgespült oder mit einem Shampoo ausgewaschen wird.

Ein dritter Gegenstand der Erfindung ist die Verwendung eines Mittels gemäß einem der Ansprüche 1 bis 23 zur Färbung keratinischer Fasern.

Die folgenden Beispiele sollen den Gegenstand der vorliegenden Anmeldung verdeutlichen, ohne ihn jedoch darauf zu beschränken.

### Beispiele

### 1. Zusammensetzung der Färbecreme

Es wurde zunächst eine Cremebasis folgender Zusammensetzung hergestellt (alle Angaben beziehen sich, soweit nicht anders vermerkt, auf die Massenteile in g):

| | |
|---|---|
| Talgfettalkohol | 17,0 |
| Lorol^{®}techn.¹ | 4,0 |
| Texapon^{®}N 28² | 40,0 |
| Dehyton^{®}K³ | 25,0 |
| Eumulgin^{®}B2⁴ | 1,5 |
| Destilliertes Wasser | 12,5 |

| | |
|---|---|
| C₁₂₋₁₈-Fettalkohol (Cognis) ² Natriumlaurylethersulfat (ca. 28% Aktivsubstanz; INCI-Bezeichnung: Sodium Laureth Sulfate) (Cognis) ³ Fettsäureamid-Derivat mit Betainstruktur der Formel R-CONH(CH₂)₃N⁺(CH₃)₂CH₂COO⁻ (ca. 30 % Aktivsubstanz; INCI-Bezeichnung: Cocoamidopropyl Betaine) (Cognis) ⁴ Cetylstearylalkohol mit ca. 20 Mol EO (INCI-Bezeichnung: Ceteareth-20) (Cognis) | |

Auf Basis dieser Creme wurde dann folgende Haarfärbeemulsion hergestellt:

| | |
|---|---|
| Cremebasis | 50,0 |
| Entwicklerkomponente | 7,5 mmol |
| Kupplerkomponente | 7,5 mmol |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3%iger Wasserstoffperoxidlösung als Oxidationslösung durchgeführt. Hierzu wurden 100 g der Emulsion mit 50 g Wasserstoffperoxidlösung (3%ig) versetzt und vermischt.

Die Färbecreme wurde auf ca. 5 cm lange Strähnen standardisierten, zu 90 % ergrauten, aber nicht besonders vorbehandelten Menschenhaares aufgetragen und dort 30 Minuten bei 32°C belassen. Nach Beendigung des Färbeprozesses wurde das Haar gespült, mit einem üblichen Haarwaschmittel gewaschen und anschließend getrocknet.

### 2. Ausfärbungsbeispiele

### a)

Es wurden die folgenden Kuppler- und Entwicklerkombinationen gewählt; die Ergebnisse der Ausfärbungen sind der folgenden Tabelle zu entnehmen:

### 1. Entwicklerkomponente:

Bis-(5-amino-2-hydroxyphenyl)-methan-dihydrochlorid (**E**)

### 2. Entwicklerkomponente

- 4,5-Diamino-1-(2'-hydroxyethyl)-pyrazoldisulfat (**E'1**)
- 4-Hydroxy-2,5,6-triaminopyrimidinsulfat (**E'2**)
- 2-Dimethylamino-4,5,6-triaminopyrimidinsulfat (**E'3**)
- 2,4,5,6-Tetraaminopyrimidinsulfat (**E'4**)

### Kupplerkomponenten:

- 2-Chlor-6-methyl-3-aminophenol (**K1**)
- m-Aminophenol (**K2**)
- 1,3-Bis-(2`,4`-diaminophenoxy)-propan-tetrahydrochlorid (**K3**)
- 2,7-Dihydroxynaphthalin (**K4**)
- 2-Amino-3-hydroxypyridin (**K5**)
- Resorcin (**K6**)

| **Entwicklerkomponenten** | **Kupplerkomponente** | **Nuance des gefärbten Haares** |
|---|---|---|
| **E + E'1** | **K1** | granatbraun |
| **E + E'1** | **K2** | cuba |
| **E + E'1** | **K3** | portweinrot |
| **E + E'2** | **K4** | gelbbraun |
| **E + E'3** | **-** | rotbraun |
| **E + E'4** | **K5** | kamelbraun |
| **E + E'1** | **K6** | madeirabraun |
| **E + E'1** | **K5** | rotbraun |

### b) Weiterhin wurden die Haarsträhnen mit den folgenden Rezepturen ausgefärbt:

### Rezeptur 1 / Farbresultat: portweinrot

### (Angaben in Gew.-%)

| | |
|---|---|
| Fettalkoholgemisch C₁₂-C₁₈¹ | 10,0 |
| Texapon^{®} K14 S70C⁵ | 2,5 |
| Plantaren^{®} 1200 UP⁶ | 2,0 |
| Akypo Soft^{®} 45 NU⁷ | 12,0 |
| Eutanol^{®} G⁸ | 1,0 |
| Eumulgin^{®} B1⁹ | 0,5 |
| Eumulgin^{®} B2⁴ | 0,5 |
| Polymer W^{®} 37194 (Stockhausen)¹⁰ | 2,0 |
| Cosmedia Guar^{®} C261¹¹ | 0,2 |
| Mirapol^{®} A15 (Polyquaternium 2)¹² | 0,5 |
| Ascorbinsäure | 0,4 |
| Ammoniumsulfat | 0,5 |
| Bis-(5-amino-2-hydroxyphenyl)-methan- | 0,124 |
| dihydrochlorid | |
| p-Toluylendiaminsulfat | 1,21 |
| 4-Amino-2-aminomethylphenol-dihydre- | 0,01 |
| chlorid | |
| 4-Amino-3-methylphenol | 0,077 |
| 2,4,5,6-Tetraaminopyrimidinsulfat | 0,08 |
| 4-Hydroxy-2,5,6-triaminopyrimidin- | 0,02 |
| sulfat | |
| 4,5-Diamino-1-(2'Hydroxyethyl)- | 0,02 |
| pyrazolsulfat | |
| 2,7-Dihydroxynaphthalin | 0,035 |
| 2-Amino-3-hydroxyppyridin | 0,44 |
| 3-Amino-2-methylamino-6- | 0,002 |
| methoxypyridin | |
| 2,6-Dihydroxy-3,4-dimethylpyridin | 0,002 |
| Resorcin | 0,12 |
| 2-Methylresorcin | 0,72 |
| 3-Aminophenol | 0,007 |
| 4-Amino-2-nitro-diphenylamin-2'- | 0,05 |
| carbonsäure | |
| 2-Ethylamino-4-nitro-6-chlorphenol | 0,05 |
| Ammoniak, 25 %ig | ad pH 9,8 |
| Wasser | ad 100 % |

### Rezeptur 2 / Farbresultat: violettbraun

### (Angaben in Gew.-%)

| | |
|---|---|
| Fettalkoholgemisch C₁₂-C₁₈¹ | 8,0 |
| Texapon^{®} NSO¹³ | 2,0 |
| Dehyton^{®} K³ | 1,0 |
| Kaliumoleat | 2,0 |
| Kaliumisostearat | 2,0 |
| Myristinsäure | 1,0 |
| Eumulgin^{®} B2⁴ | 0,5 |
| Westvaco Diacid H 240, K-Salz¹⁸ | 2,0 |
| Merquat^{®} 550 (Polyquaternium 7)¹⁴ | 0,2 |
| Luviquat^{®} FC 370 (Polyquaternium 16)¹⁵ | 0,1 |
| Merquat^{®} 280 (Polyquaternium 22)¹⁶ | 0,1 |
| Gafquat^{®} HS-100 (Polyquaternium 28)¹⁷ | 0,1 |
| Ascorbinsäure | 0,4 |
| Ammoniumsulfat | 0,5 |
| Bis-(5-amino-2-hydroxyphenyl)- | 0,124 |
| methan-dihydrochlorid | |
| p-Toluylendiaminsulfat | 0,40 |
| 2-(2'-Hydroxyethyl)-p-phenylen- | 0,10 |
| diamin-sulfat | |
| 2-Aminomethyl-4-aminophenol | 0,01 |
| 4,5-Diamino-1-(2`Hydroxyethyl- | 0,24 |
| pyrazol)-sulfat | |
| 2,7-Dihydroxynaphthalin | 0,10 |
| 5-Amino-2-methylphenol | 0,15 |
| 5-(2'-Hydroxyethyl)amino- | 0,44 |
| 3-methylphenol | |
| 5-Amino-4-chlor-2-methylphenol | 0,1 |
| Resorcin | 0,04 |
| 2-Methylresorcin | 0,08 |
| 4-Chlorresorcin | 0,05 |
| Ammoniak, 25 %ig | ad pH 9,8 |
| Wasser | ad 100 % |

### Rezeptur 3 / Farbresultat: englischrot

### (Angaben in Gew.-%)

| | |
|---|---|
| Fettalkoholgemisch C₁₂-C₁₈¹ | 8,0 |
| Texapon^{®} NSO¹³ | 2,0 |
| Dehyton^{®} K³ | 1,0 |
| Kaliumoleat | 2,0 |
| Kaliumisostearat | 2,0 |
| Myristinsäure | 1,0 |
| Eumulgin^{®} B2⁴ | 0,5 |
| Westvaco Diacid H 240, K-Salz¹⁸ | 2,0 |
| Merquat^{®} 550 (Polyquaternium 7)¹⁴ | 0,2 |
| Luviquat^{®} FC 370 (Polyquaternium 16)¹⁵ | 0,1 |
| Merquat^{®} 280 (Polyquaternium 22)¹⁶ | 0,1 |
| Gatquat^{®} HS-100 (Polyquaternium 28)¹⁷ | 0,1 |
| Ascorbinsäure | 0,4 |
| Ammoniumsulfat | 0,5 |
| Bis-(5-amino-2-hydroxyphenyl)- | 0,030 |
| methan-dihydrochlorid | |
| 4-Amino-2-{(4-[5'-Amino-2'-hydroxy- | 0,012 |
| phenyl)-methyl]piperazinyl)methyl}- | |
| phenol-tetrahydrochlorid | |
| p-Phenylendiamindihydrochlorid | 0,10 |
| 4-Amino-2-aminomethylphenol- | 0,3 |
| dihydro-chlorid | |
| 4-Amino-2-((diethylamino)methyl)- | 0,8 |
| phenoldihydrochlorid | |
| 4-Amino-3-methylphenol | 0,011 |
| 2,4,5,6-Tetraaminopyrimidinsulfat | 0,80 |
| 4-Hydroxy-2,5,6-triaminopyrimidin- | 0,08 |
| sulfat | |
| 2,7-Dihydroxynaphthalin | 0,33 |
| 5-(2'-Hydroxyethyl)amino- | 0,015 |
| 2-methylphenol | |
| 5-Amino-4-chlor-2-methylphenol | 0,030 |
| Resorcin | 0,044 |
| 2-Methylresorcin | 0,25 |
| 3-Aminophenol | 0,004 |
| 4-Amino-2-nitro-diphenylamin-2'- | 0,15 |
| carbonsäure | |
| 4-Amino-3-nitrophenol | 0,25 |
| Ammoniak, 25 %ig | ad pH 9,8 |
| Wasser | ad 100 % |

| | |
|---|---|
| ⁵ Natriumlaurylmyristyllaurelethersulfat (68-73% Aktivsubstanz); INCI-Bezeichnung: Sodium Myreth Sulfate; (Cognis) ⁶ C₁₂-C₁₆-Fettalkohol-1,4-glucosid (50-53% Aktivsubstanz); INCI-Bezeichnung: Lauryl Glucoside; (Cognis) ⁷ C₁₂-C₁₄-Fettalkohol-4.5-EO-Essigsäure-Natrium-Salz (21% Aktivsubstanz); INCI-Bezeichnung: Sodium Laureth-6 Carboxylate; (KAO) ⁸ 2-Octyldecanol; INCI-Bezeichnung: Octyl Decanol; (Cognis) ⁹ Cetearylalkohol mit 12 Mol EO; INCI-Bezeichnung: Ceteareth-12; (Cognis) ¹⁰ Acrylsäure-Natrium-Salz-Acrylamidopropyltrimethylammoniumchlorid-Copolymer; INCI-Bezeichnung: Acrylamidopropyltrimoniumchloride/Acrylates Copolymer; (Stockhausen) ¹¹ Guarhydroxypropyltrimethylammoniumchlorid; INCI-Bezei chnung: Guar Hydroxypropyltrimonium Chloride; (Cognis) ¹² Poly[N-3-(dimethylammonium)propyl]-N'-[3-ethylenoxyethylen-dimethylammonium) propyl]-harnstoff-dichlorid; INCI-Bezeichnung: Polyquaternium-2; (Rhodia) ¹³ Natriumlaurylethersulfat (ca. 27% Aktivsubstanz; INCI-Bezeichnung: Sodium Laureth Sulfate) (Cognis) ¹⁴ Dimethyldiallylammoniumchlorid-Acrylamid-Copolymer (8-9% Aktivsubstanz); INCI-Bezeichnung: Polyquaternium-7 (Chemviron) ¹⁵ Vinylimidazoliummethochlorid-Vinylpyrrolidon-Copolymerisat; INCI-Bezeichnung: Polyquaternium-16) (BASF) ¹⁶ Dimethyldiallylammoniumchlorid-Acrylsäure-Copolymer (35% Aktivsubstanz); INCI-Bezeichnung: Polyquaternium-22 (Chemviron) ¹⁷ Vinylpyrrolidon-Methacrylamidopropyltrimethylammoniumchlorid-Copolymer (19-21% Aktivsubstanz); INCI-Bezeichnung: Polyquaternium-28 (General Anilin Firm Corp.) ¹⁸ 4-Hexyl-5(6)-carboxy-2-cyclohexen-1-octansäure, Kalium-Salz; (Westvaco Chemicals) | |

## Patentansprüche

1. Mittel zum Färben keratinischer Fasern, insbesondere von menschlichen Haaren, **dadurch gekennzeichnet, dass** es in einem zum Färben geeigneten Medium als Oxidationsfarbstoffvorprodukte eine Kombination aus
a) Bis-(5-amino-2-hydroxyphenyl)-methan oder einem seiner physiologisch verträglichen Salze als Entwicklerkomponente und
b) mindestens einer weiteren, heterocyclischen Entwicklerkomponente, ausgewählt aus der Gruppe der
- Pyrimidinderivate,
- Pyrazolderivate und der
- Pyrazol-Pyrimidin-Derivate,
sowie deren physiologisch verträglichen Salzen
enhält.

2. Mittel nach Anspruch 1, **dadurch gekennzeichnet, dass** das Pyrimidinderivat ausgewählt ist aus Verbindungen der Formel (II) 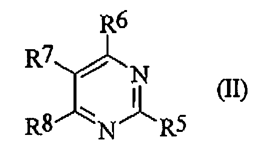 in der
- R⁵ steht für eine Hydroxygruppe oder eine Gruppe -NRR', in der R und R' unabhängig voneinander stehen für Wasserstoff, eine Hydroxyalkyl- oder Alkylgruppe, jeweils mit 1 bis 4 Kohlenstoffatomen, oder sie bilden mit dem sie tragenden Stickstoffatom ein heterocyclisches Ringsystem,
- R⁶, R⁷ und R⁸ stehen unabhängig voneinander für eine Hydroxygruppe, eine Aminogruppe oder eine C₁-C₄-Dialkylaminogmppe,
sowie deren physiologisch verträgliche Salze,

3. Mittel nach Anspruch 2, **dadurch gekennzeichnet, dass** das Pyrimidinderivat der Formel (II) ausgewählt ist aus 4-Hydroxy-2,5,6-triaminopyrimidin, 5-Hydroxy-2,4,6-triaminopyrimidin, 2-Dimethylainino-4,5,6-triaminopyrimidin und 2,4,5,6-Tetraaminopyrimidin sowie deren physiologisch verträglichen Salzen.

4. Mittel nach einem der Ansprüche 1 bis 3, **dadurch gekennzeichnet, dass** das Pyrazolderivat ausgewählt ist aus Verbindungen der Formel (III) 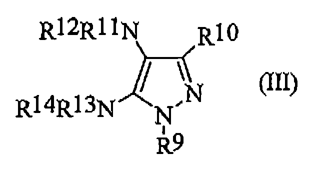 in der
- R⁹, R¹¹, R¹², R¹³ und R¹⁴ unabhängig voneinander stehen für Wasserstoff, eine geradkettige oder verzweigte eine C₁-C₆-Alkylgruppe, eine C₁-C₄-Mono- oder eine C₂-C₄-Polyhydroxyalkylgruppe, eine C₂-C₄-Aminoalkylgruppe, eine Phenylgruppe, die gegebenenfalls mit einer Nitrogruppe, einer Trifluormethylgruppe, oder einer Aminogruppe substituiert ist, eine Benzylgruppe, die gegebenenfalls mit einem Halogenatom, einer C₁-C₄-Alkylgruppe, einer Methylendioxygruppe oder einer Aminogruppe substituiert ist, eine Pyridylgruppe, eine Thienylgruppe, eine Furylgruppe oder eine Gruppe 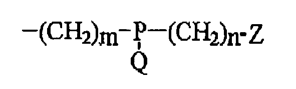 in der m und n unabhängig voneinander ganze Zahlen im Bereich von 1 bis 3 darstellen, P steht für Sauerstoff oder eine Gruppe NH-, Q steht für Wasserstoff oder eine Methylgruppe und Z steht für eine Methylgruppe, eine Gruppe -OR oder -NRR', wobei R und R' unabhängig voneinander Wasserstoff, eine Methyl- oder eine Ethylgruppe sein können und für den Fall, dass R¹³ Wasserstoff ist, R¹⁴ auch C₁-C₄-Alkylamino bedeuten kann und
- R¹⁰ steht für Wasserstoff, eine geradkettige oder verzweigte C₁-C₆-Alkylgruppe, eine C₂-C₄-Hydroxyalkylgruppe, eine C₁-C₄-Ammoalkylgruppe, eine N-C₁-C₄-Alkylamino-C₁-C₄-alkylgruppe, eine C₁-C₄-Alkoxymethylgmppe, eine Phenylgruppe, die gegebenenfalls mit einem Halogenatom, einer C₁-C₄-Alkylgruppe, einer C₁-C₄-Alkoxygruppe, einer Aminogruppe, einer Trifluormethylgruppe, einer C₁-C₄-Aminoalkylgruppe oder einer Nitrogruppe substituiert ist, einen Heterocyclus, der unter Thiophen, Furan und Pyridin ausgewählt ist, oder eine Gruppe -(CH₂)ₚ-O-(CH₂)_{q}-OR', worin R' für Wasserstoff oder eine Methylgruppe steht und p und q ganze Zahlen sind, die unabhängig voneinander im Bereich von 1 bis 3 liegen, oder für Wasserstoff mit der Maßgabe, dass dann R₉ von einer unsubstituierten oder substituierten Benzylgruppe verschieden ist.

5. Mittel nach Anspruch 4, **dadurch gekennzeichnet, dass** das Pyrazolderivat ausgewählt ist aus 4,5-Diaminopyrazol, 4,5-Diamino-1-(2'-hydroxyethyl)-pyrazol, 4,5-Diamino-1,3-dimethyl-pyrazol, 4,5-Diamino-3-methyl-1-phenyl-pyrazol, 4,5-Diamino-1-methyl-3-phenyl-pyrazol, 1-Benzyl-4,5-Diamino-3-methyl-pyrazol, 4,5-Diamino-1-tert,-butyl-3-methyl-pyrazol, 4,5-Diamino-1-(2'-hydroxyethyl)-3-methyl-pyrazol, 4,5-Diamino-1-ethyl-3-methyl-pyrazol, 4,5-Diamino-1-ethyl-3-hydroxymethyl-pyrazol, 4,5-Diamino-3-hydroxymethyl-1-methyl-pyrazol, 4,5-Diamino-3-hydroxymethyl-1-isopropyl-pyrazol oder einem der physiologisch verträglichen Salze dieser Verbindungen.

6. Mittel nach Anspruch 5, **dadurch gekennzeichnet, dass** das Pyrazolderivat 4,5-Diamino-1-(2'-hydroxyethyl)-pyrazol oder eines seiner physiologisch verträglichen Salze ist.

7. Mittel nach einem der Ansprüche 1 bis 6, **dadurch gekennzeichnet, dass** das Pyrazol-Pyrimidin-Derivat ausgewählt ist aus Verbindungen der Formel (IV) 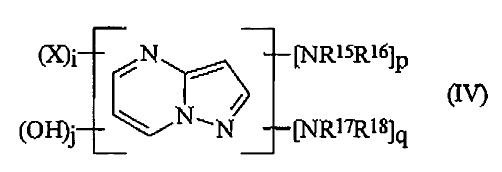 wobei:
- R¹⁵, R¹⁶, R¹⁷ und R¹⁸ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die Reste X stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- j hat den Wert 0 oder 1,
mit der Maßgabe, dass
- die Summe aus p + q ungleich 0 ist,
- wenn p + q gleich 2 ist, n den Wert 0 hat, und die Gruppen NR¹⁵R¹⁶ und NR¹⁷R¹⁸ belegen die Positionen (2,3); (5,6); (6,7); (3,5) oder (3,7);
- wenn p + q gleich 1 ist, n den Wert 1 hat, und die Gruppen NR¹⁵R¹⁶ (oder N-R¹⁷R¹⁸) und die Gruppe OH belegen die Positionen (2,3); (5,6); (6,7); (3,5) oder (3,7).

8. Mittel nach Anspruch 7, **dadurch gekennzeichnet, dass** das Pyrazol-Pyrimidinderivat ausgewählt ist aus Pyrazol-[1,5-a]-pyrimidin-3,7-diamin, 2,5-Dimethyl-pyrazol-[1,5-a]-pyrimidin-3,7-diamin, Pyrazol-[1,5-a]-pylimidin-3,5-diarnin, 2,7-Dimethyl-pyrazol-[1,5-a]-pyrimidin-3,5-diamin, 3-Aminopyrazol-[1,5-a]-pyrimidin-7-ol, 3-Aminopyrazol-[1,5-a]-pyrimidin-5-ol, 2-(3-Aminopyrazol-[1,5-a]-pyrimidin-7-ylamino)-ethanal, 2-(7-Aminopyrazol-[1,5-a]-pyrimidin-3-ylamino)-ethanol, 2-[(3-Aminopyrazol-[1,5-a]-pyrimidin-7-yl)-(2-hydroxy-ethyl)-amino]-ethanol, 2-[(7-Aminopyrazol-[1,5-a]-pyrimidin-3-yl)-(2-hydroxy-ethyl)-amino]-ethanol, 5,6-Dimethylpyrazol-[1,5-a]-pyrimidin-3,7-diamin, 2,6-Dimethylpyrazol-[1,5-a]-pyrimidin-3,7-diamin und 3-Amino-7-dimethylamino-2,5-dimethylpyrazol-[1,5-a]-pyrimidin oder einem der physiologisch verträglichen Salze dieser Verbindungen.

9. Mittel nach einem der Ansprüche 1 bis 8, **dadurch gekennzeichnet, dass** es mindestens noch eine weitere Entwicklerkomponente enthält.

10. Mittel nach Anspruch 9, **dadurch gekennzeichnet, dass** die weitere Entwicklerkomponente, ausgewählt ist aus p-Phenylendiamin, p-Toluylendiamin, p-Aminophenol, 1-(2'-Hydroxyethyl)-2,5-diaminobenzol, 1,10- Bis-(2',5' - diaminophenyl)-1,4,7,10-tetraoxydecan, N,N-Bis-(2`-hydroxyethyl)-p-phenylendiamin, 4-Amino- 3-methylphenol, 4-Amino-2-((diethylamino)-methyl)-phenol, 4-Amino-2,6-dichlorphenol oder 2-Aminomethyl-4-aminophenol sowie den physiologisch verträglichen Salzen dieser Verbindungen.

11. Mittel nach einem der Ansprüche 1 bis 10, **dadurch gekennzeichnet, dass** es mindestens eine Kupplerkomponente enthält.

12. Mittel nach Anspruch 11, **dadurch gekennzeichnet, dass** die Kupplerkomponente, ausgewählt ist aus 1-Naphthol, 1,5-Dihydroxynaphthalin, 1,7-Dihydroxynaohthalin, 2,7-Dihydroxynaphthalin, 3-Aminophenol, 5-Amino-2-methylphenol, 5-(2'-Hydroxyethylamino)-3-methylphenol, 2,4-Dichlor-3-aminophenol, 2-Chlor-6-methyl-3-aminophenol, 2-Methyl-4-chlor-5-aminophenol, Resorcin, Resorcinmonomethylester, 2-Chlorresorcin, 4-Chlorresorcin, 2-Methyhresorcin, 5-Methylresorcin, 2,5-Dimethylresorcin, m-Phenylendiamin, 2-Methylamino-3-amino-6-methoxypyridin, 2,4-Diaminophenoxyethanol, 1,3-Bis-(2',4'-diaminophenoxy)-propan, 2-Amino-3-hydroxypyridin oder 2,6-Dihydroxy-3,4-dimethylpyridin sowie aus den physiologisch verträglichen Salzen dieser Verbindungen.

13. Mittel nach einem der Ansprüche 1 bis 12, **dadurch gekennzeichnet, dass** es weiterhin Vorstufen naturanaloger Farbstoffe, wie Indol- und Indolin-Derivate, enthält.

14. Mittel nach einem der Ansprüche 1 bis 13, **dadurch gekennzeichnet, dass** die Entwicklerkomponenten in einer Menge von 0,005 bis 20 Gew.-%, vorzugsweise 0,1 bis 5 Gew.%, und die Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

15. Mittel nach einem der Ansprüche 1 bis 14, **dadurch gekennzeichnet, dass** weiterhin mindestens ein direktziehender Farbstoff enthalten ist.

16. Verfahren zum Färben von Keratinfasern, **dadurch gekennzeichnet, dass** ein Mittel zum Färben von Keratinfasern nach einem der Ansprüche 1 bis 15 in einer geeigneten kosmetischen Zubereitung auf die zu färbenden Fasern aufgebracht und nach einer Einwirkungszeit wieder ausgespült oder mit einem Shampoo ausgewaschen wird.

17. Verwendung eines Mittels gemäß einem der Ansprüche 1 bis 15 zur Färbung keratinischer Fasern.

## Claims

1. An agent for dyeing keratin fibres, in particular human hair, **characterised in that**, in a medium suitable for dyeing, it contains as oxidation dye precursors a combination of
a) bis-(5-amino-2-hydroxyphenyl)-methane or one of the physiologically acceptable salts thereof as developer component and
b) at least one further, heterocyclic developer component, selected from the group of
- pyrimidine derivatives,
- pyrazole derivatives and
- pyrazole-pyrimidine derivatives,
and the physiologically acceptable salts thereof.

2. An agent according to claim 1, **characterised in that** the pyrimidine derivative is selected from compounds of the formula (II) 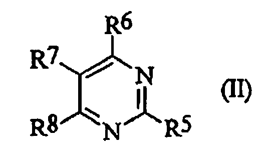 in which
- R⁵ denotes a hydroxyl group, or a group -NRR', in which R and R' mutually independently denote hydrogen, a hydroxyalkyl or alkyl group, in each case with 1 to 4 carbon atoms, or they form a heterocyclic ring system with the nitrogen atom bearing them,
- R⁶, R⁷ and R⁸ mutually independently denote a hydroxyl group, an amino group or a C₁-C₄ dialkylamino group,
and the physiologically acceptable salts thereof.

3. An agent according to claim 2, **characterised in that** the pyrimidine derivative of the formula (II) is selected from 4-hydroxy-2,5,6-triaminopyrimidine, 5-hydroxy-2,4,6-triaminopyrimidine, 2-dimethylamino-4,5,6-triaminopyrimidine and 2,4,5,6-tetraaminopyrimidine and the physiologically acceptable salts thereof.

4. An agent according to any one of claims 1 to 3, **characterised in that** the pyrazole derivative is selected from compounds of the formula (III) 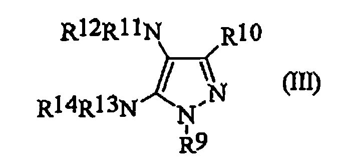 in which
- R⁹, R¹¹, R¹² , R¹³ and R¹⁴ mutually independently denote hydrogen, a straight-chain or branched C₁-C₆ alkyl group, a C₁-C₄ mono- or a C₂-C₄ polyhydroxyalkyl group, a C₂-C₄ aminoalkyl group, a phenyl group, which is optionally substituted with a nitro group, a trifluoromethyl group, or an amino group, a benzyl group, which is optionally substituted with a halogen atom, a C₁-C₄ alkyl group, a methylenedioxy group or an amino group, a pyridyl group, a thienyl group, a furyl group or a group 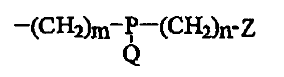
in which m and n mutually independently represent integers in the range from 1 to 3, P denotes oxygen or a group NH-, Q denotes hydrogen or a methyl group and Z denotes a methyl group, a group -OR or -NRR', wherein R and R' may mutually independently be hydrogen, a methyl or an ethyl group and, in the event that R¹³ is hydrogen, R¹⁴ may also mean C₁-C₄ alkylamino and
- R¹⁰ denotes hydrogen, a straight-chain or branched C₁-C₆ alkyl group, a C₂-C₄ hydroxyalkyl group, a C₁-C₄ aminoalkyl group, an N-C₁-C₄-alkylamino-C₁-C₄-alkyl group, a C₁-C₄ alkoxymethyl group, a phenyl group, which is optionally substituted with a halogen atom, a C₁-C₄ alkyl group, a C₁-C₄ alkoxy group, an amino group, a trifluoromethyl group, a C₁-C₄ aminoalkyl group or a nitro group, a heterocycle, which is selected from among thiophene, furan and pyridine, or a group -(CH₂)ₚ-O-(CH₂)_{q}-OR' in which R' denotes hydrogen or a methyl group and p and q are integers, which are mutually independently in the range from 1 to 3, or denotes hydrogen providing that R⁹ is then different from an unsubstituted or substituted benzyl group.

5. An agent according to claim 4, **characterised in that** the pyrazole derivative is selected from 4,5-diaminopyrazole, 4,5-diamino-1-(2'-hydroxyethyl)pyrazole, 4,5-diamino-1 ,3-dimethylpyrazole, 4,5-diamino-3-methyl-1-phenylpyrazole, 4,5-diamino-1-methyl-3-phenylpyrazole, 1-benzyl-4,5-diamino-3-methylpyrazole, 4,5-diamino-1-tert.-butyl-3-methylpyrazole, 4,5-diamino-1-(2'-hydroxyethyl)-3-methylpyrazole, 4,5-diamino-1-ethyl-3-methylpyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, or one of the physiologically acceptable salts of these compounds.

6. An agent according to claim 5, **characterised in that** the pyrazole derivative is 4,5-diamino-1-(2'-hydroxyethyl)-pyrazole or one of the physiologically acceptable salts thereof.

7. An agent according to any one of claims 1 to 6, **characterised in that** the pyrazole-pyrimidine derivative is selected from compounds of the formula (IV) 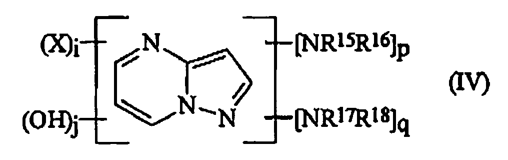 wherein:
- R¹⁵, R¹⁶ , R¹⁷ and R¹⁸ mutually independently denote a hydrogen atom, a C₁ to C₄ alkyl residue, an aryl residue, a C₁ bis C₄ hydroxyalkyl residue, a C₂ to C₄ polyhydroxyalkyl residue, a (C₁- to C₄)-alkoxy-(C₁- to C₄)-alkyl residue, a C₁ to C₄ aminoalkyl residue, which may optionally be protected by an acetyl ureide or a sulfonyl residue, a (C₁- to C₄)-alkylamino-(C₁- to C₄)-alkyl residue, a di-[(C₁- to C₄)-alkyl]-(C₁- to C₄)-aminoalkyl residue, wherein the dialkyl residues optionally form a carbocycle or a heterocycle with 5 or 6 chain links, a C₁- to C₄-hydroxyalkyl- or a di-(C₁- to C₄)-[hydroxyalkyl]-(C₁- to C₄)-aminoalkyl residue,
- the residues X mutually independently denote a hydrogen atom, a C₁ to C₄ alkyl residue, an aryl residue, a C₁ bis C₄ hydroxyalkyl residue, a C₂ to C₄ polyhydroxyalkyl residue, a C₁ to C₄ aminoalkyl residue, a (C₁- to C₄)-alkylamino-(C₁- to C₄)-alkyl residue, a di-[(C₁- to C₄)-alkyl]-(C₁- to C₄)-aminoalkyl residue, wherein the dialkyl residues optionally form a carbocycle or a heterocycle with 5 or 6 chain links, a C₁- to C₄-hydroxyalkyl- or a di-(C₁- to C₄-hydroxyalkyl)-aminoalkyl residue, an amino residue, a C₁- to C₄-alkyl- or di-(C₁- to C₄-hydroxyalkyl)amino residue, a halogen atom, a carboxylic acid group or a sulfonic acid group,
- i has the value 0, 1, 2 or 3,
- p has the value 0 or 1,
- q has the value 0 or 1 and
- j has the value 0 or 1,
providing that
- the sum of p + q does not equal 0,
- if p + q is equal to 2, n has the value 0, and the groups NR¹⁵R¹⁶ and NR¹⁷R¹⁸ occupy positions (2,3); (5,6); (6,7); (3,5) or (3,7);
- if p + q is equal to 1, n has the value 1, and the groups NR¹⁵R¹⁶ (or NR¹⁷R¹⁸) and the group OH occupy positions (2,3); (5,6); (6,7); (3,5) or (3,7).

8. An agent according to claim 7, **characterised in that** the pyrazole-pyrimidine derivative is selected from pyrazole-[1,5a]-pyrimidine-3,7-diamine, 2,5-dimethylpyrazole-[1,5-a]-pyrimidine-3,7-diamine, pyrazole-[1,5a]-pyrimidine-3,5-diamine, 2,7-dimethylpyrazole-[1,5-a]-pyrimidine-3,5-diamine, 3-aminopyrazole-[1,5-a]-pyrimidin-7-ol, 3-aminopyrazole-[1,5-a]-pyrimidin-5-ol, 2-(3-aminopyrazole-[1,5-a]-pyrimidin-7-ylamino)-ethanol, 2-(7-aminopyrazole-[1,5-a]-pyrimidin-3-ylamino)-ethanol, 2-[(3-aminopyrazole-[1,5-a]-pyrimidin-7-yl)-(2-hydroxyethyl)-amino]-ethanol, 2-[(7-aminopyrazole-[1,5-a]-pyrimidin-3-yl)-(2-hydroxyethyl)-amino]-ethanol, 5,6-dimethylpyrazole-[1,5-a]-pyrimidine-3,7-diamine, 2,6-dimethylpyrazole-[1,5-a]-pyrimidine-3,7-diamine and 3-amino-7-dimethylamino-2,5-dimethylpyrazole-[1,5-a]-pyrimidine or one of the physiologically acceptable salts of these compounds.

9. An agent according to any one of claims 1 to 8, **characterised in that** it contains at least one further developer component.

10. An agent according to claim 9, **characterised in that** the further developer component is selected from p-phenylenediamine, p-toluylenediamine, p-aminophenol, 1-(2'-hydroxyethyl)-2,5-diaminobenzene, 1,10-bis-(2',5'-diaminophenyl)-1,4,7,10-tetraoxydecane, N,N-bis-(2'-hydroxyethyl)-p-phenylenediamine, 4-amino-3-methylphenol, 4-amino-2-((diethylamino)-methyl)-phenol, 4-amino-2,6-dichlorophenol or 2-aminomethyl-4-aminophenol and the physiologically acceptable salts of these compounds.

11. An agent according to any one of claims 1 to 10, **characterised in that** it contains at least one coupler component.

12. An agent according to claim 11, **characterised in that** the coupler component is selected from 1-naphthol, 1,5-dihydroxynaphthalene, 1,7-dihydroxynaphthalene, 2,7-dihydroxynaphthalene, 3-aminophenol, 5-amino-2-methylphenol, 5-(2'-hydroxyethylamino)-3-methylphenol, 2,4-dichloro-3-aminophenol, 2-chloro-6-methyl-3-aminophenol, 2-methyl-4-chloro-5-aminophenol, resorcinol, resorcinol monomethyl ester, 2-chlororesorcinol, 4-chlororesorcinol, 2-methylresorcinol, 5-methylresorcinol, 2,5-dimethylresorcinol, m-phenylenediamine, 2-methylamino-3-amino-6-methoxypyridine, 2,4-diaminophenoxyethanol, 1,3-bis-(2',4'-diaminophenoxy)propane, 2-amino-3-hydroxypyridine, or 2,6-dihydroxy-3,4-dimethylpyridine and from the physiologically acceptable salts of these compounds.

13. An agent according to any one of claims 1 to 12, **characterised in that** it furthermore contains precursors of nature-analogous dyes, such as indole and indoline derivatives.

14. An agent according to any one of claims 1 to 13, **characterised in that** the developer components are present in a quantity of 0.005 to 20 wt.%, preferably 0.1 to 5 wt.%, and the coupler components in a quantity of 0.005 to 20 wt.%, preferably 0.1 to 5 wt.%, in each case relative to the entire oxidation dye preparation.

15. An agent according to any one of claims 1 to 14, **characterised in that** at least one direct dye is furthermore present.

16. A method for dyeing keratin fibres, **characterised in that** an agent for dyeing keratin fibres according to any one of claims 1 to 15 in a suitable cosmetic preparation is applied onto the fibres to be dyed and, after a period of exposure, is rinsed out again or washed out with a shampoo.

17. Use of an agent according to any one of claims 1 to 15 for dyeing keratin fibres.

## Revendications

1. Agent pour la teinture de fibres kératiniques, en particulier de cheveux humains, **caractérisé en ce qu'**il contient, dans un milieu approprié pour la teinture, comme précurseurs de colorants par oxydation, une combinaison
a) de bis-(5-amino-2-hydroxyphényl)-méthane ou d'un de ses sels physiologiquement acceptables comme composant de développement et
b) d'au moins un autre composant de développement hétérocyclique, choisi dans le groupe des
- dérivés de pyrimidine,
- dérivés de pyrazole et
- dérivés de pyrazole-pyrimidine,
ainsi que leurs sels physiologiquement acceptables.

2. Agent selon la revendication 1, **caractérisé en ce que** le dérivé de pyrimidine est choisi dans les composés de formule (II) 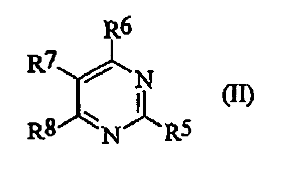 dans laquelle
- R⁵ représente un groupe hydroxy ou un groupe -NRR', où R et R' représentent, indépendamment l'un de l'autre, hydrogène, un groupe hydroxyalkyle ou alkyle, comprenant à chaque fois 1 à 4 atomes de carbone ou ils forment avec l'atome d'azote qui les porte un système hétérocyclique,
- R⁶, R⁷ et R⁸ représentent, indépendamment l'un de l'autre, un groupe hydroxy, un groupe amino ou un groupe C₁-C₄-dialkylamino,
ainsi que leurs sels physiologiquement acceptables.

3. Agent selon la revendication 2, **caractérisé en ce que** le dérivé de pyrimidine de formule (II) est choisi parmi la 4-hydroxy-2,5,6-triaminopyrimidine, la 5-hydroxy-2,4,6-triaminopyrimidine, la 2-diméthylamino-4,5,6-triaminopyrimidine et la 2,4,5,6-tétraaminopyrimidine ainsi que leurs sels physiologiquement acceptables.

4. Agent selon l'une quelconque des revendications 1 à 3, **caractérisé en ce que** le dérivé de pyrazole est choisi dans les composés de formule (III) 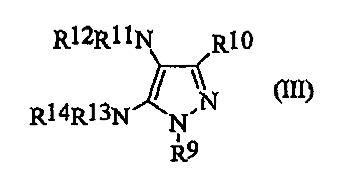 dans laquelle
R⁹, R¹¹, R¹² , R¹³ et R¹⁴ représentent, indépendamment l'un de l'autre, hydrogène, un groupe C₁-C₆-alkyle linéaire ou ramifié, un groupe C₁-C₄-monohydroxyalkyle ou C₂-C₄-polyhydroxyalkyle, un groupe C₂-C₄-aminoalkyle, un groupe phényle, qui est le cas échéant substitué par un groupe nitro, un groupe trifluorométhyle, ou un groupe amino, un groupe benzyle, qui est le cas échéant substitué par un atome d'halogène, un groupe C₁-C₄-alkyle, un groupe méthylènedioxy ou un groupe amino, un groupe pyridiyle, un groupe thiényle, un groupe furyle ou un groupe 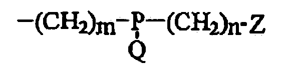 où m et n représentent, indépendamment l'un de l'autre, des nombres entiers dans la plage de 1 à 3, P représente oxygène ou un groupe NH-, Q représente hydrogène ou un groupe méthyle et Z représente un groupe méthyle, un groupe -OR ou -NRR', où R et R' peuvent représenter, indépendamment l'un de l'autre hydrogène, un groupe méthyle ou un groupe éthyle et, pour le cas où R¹³ représente hydrogène, R¹⁴ peut également signifier C₁-C₄-alkylamino et
R¹⁰ représente hydrogène, un groupe C₁-C₆-alkyle linéaire ou ramifié, un groupe C₂-C₄-hydroxyalkyle, un groupe C₁-C₄-aminoalkyle, un groupe N-C₁-C₄-alkylamino-C₁-C₄-alkyle, un groupe C₁-C₄-alcoxyméthyle, un groupe phényle, qui est le cas échéant substitué par un atome d'halogène, un groupe C₁-C₄-alkyle, un groupe C₁-C₄-alcoxy, un groupe amino, un groupe trifluorométhyle, un groupe C₁-C₄-aminoalkyle ou un groupe nitro, un hétérocycle qui est choisi parmi le thiophène, le furanne et la pyridine, ou un groupe -(CH₂)ₚ-O-(CH₂)_{q}-OR', où R' représente hydrogène ou un groupe méthyle et p et q sont des nombres entiers qui se situent, indépendamment l'un de l'autre, dans la plage de 1 à 3, ou hydrogène à condition que R⁹ soit alors différent d'un groupe benzyle non substitué ou substitué.

5. Agent selon la revendication 4, **caractérisé en ce que** le dérivé de pyrazole est choisi parmi le 4,5-diaminopyrazole, le 4,5-diamino-1-(2'-hydroxyéthyl)-pyrazole, le 4,5-diamino-1,3-diméthylpyrazole, le 4,5-diamino-3-méthyl-1-phénylpyrazole, le 4,5-diamino-1-méthyl-3-phénylpyrazole, le 1-benzyl-4,5-diamino-3-méthylpyrazole, le 4,5-diamino-1-tert-butyl-3-méthylpyrazole, le 4,5-diamino-1-(2'-hydroxyéthyl)-3-méthylpyrazole, le 4,5-diamino-1-éthyl-3-méthylpyrazole, le 4,5-diamino-1-éthyl-3-hydroxyméthylpyrazole, le 4,5-diamino-3-hydroxyméthyl-1-méthylpyrazole, le 4,5-diamino-3-hydroxyméthyl-1-isopropylpyrazole ou un des sels physiologiquement acceptables de ces composés.

6. Agent selon la revendication 5, **caractérisé en ce que** le dérivé de pyrazole est le 4,5-diamino-1-(2'-hydroxyéthyl)-pyrazole ou un de ses sels physiologiquement acceptables.

7. Agent selon l'une quelconque des revendications 1 à 6, **caractérisé en ce que** le dérivé de pyrazole-pyrimidine est choisi parmi les composés de formule (IV) 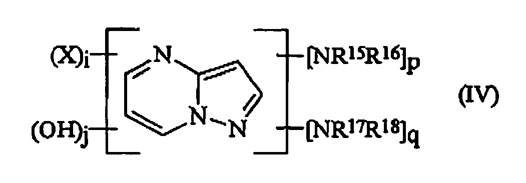 où:
- R¹⁵ , R¹⁶, R¹⁷ et R¹⁸ représentent indépendamment l'un de l'autre un atome d'hydrogène, un radical alkyle en C₁ à C₄, un radical aryle, un radical hydroxyalkyle en C₁ à C₄, un radical polyhydroxyalkyle en C₂ à C₄, un radical (alcoxy en C₁ à C₄)(alkyle en C₁ à C₄), un radical aminoalkyle en C₁ à C₄, qui peut le cas échéant être protégé par un radical acétyle, uréide ou sulfonyle, un radical (alkyle en C₁ à C₄)amino(alkyle en C₁ à C₄), un radical di[alkyle en C₁ à C₄](aminoalkyle en C₁ à C₄), où les radicaux dialkyle forment le cas échéant un cycle carboné ou un hétérocycle avec 5 ou 6 chaînons, un radical (hydroxyalkyle en C₁ à C₄)amino(alkyle en C₁ à C₄) ou un radical di[hydroxyalkyle en C₁ à C₄]amino(alkyle en C₁ à C₄),
- les radicaux X représentent indépendamment l'un de l'autre un atome d'hydrogène, un radical alkyle en C₁ à C₄, un radical aryle, un radical hydroxyalkyle en C₁ à C₄, un radical polyhydroxyalkyle en C₂ à C₄, un radical aminoalkyle en C₁ à C₄, un radical (alkyle en C₁ à C₄)amino(alkyle en C₁ à C₄), un radical di[alkyle en C₁ à C₄]amino(alkyle en C₁ à C₄) où les radicaux dialkyle forment le cas échéant un cycle carboné ou un hétérocycle avec 5 ou 6 chaînons, un radical hydroxy(alkyle en C₁ à C₄)aminoalkyle ou un radical di[hydroxyalkyle en C₁ à C₄]-aminoalkyle, un radical amino, un radical (hydroxyalkyle en C₁ à C₄)-amino ou un radical di(hydroxyalkyle en C₁ à C₄)amino, un atome d'halogène, un groupe acide carboxylique ou un groupe acide sulfonique,
- i vaut 0, 1, 2 ou 3,
- p vaut 0 ou 1,
- q vaut 0 ou 1 et
- j vaut 0 ou 1,
à condition que
- la somme de p + q soit différente de 0,
- lorsque p + q vaut 2, n vaille 0 et les groupes NR¹⁵R¹⁶ et NR¹⁷R¹⁸ occupent les positions (2,3) ; (5,6) ; (6,7) ; (3,5) ou (3,7) ;
- lorsque p + q vaut 1, n vaille 1 et les groupes NR¹⁵R¹⁶ (ou NR¹⁷R¹⁸) et le groupe OH occupent les positions (2,3) ; (5,6) ; (6,7) ; (3,5) ou (3,7).

8. Agent selon la revendication 7, **caractérisé en ce que** le dérivé de pyrazole-pyrimidine est choisi parmi la pyrazole-[1,5-a]-pyrimidine-3,7-diamine, la 2,5-diméthylpyrazole-[1,5-a]-pyrimidine-3,7-diamine, la pyrazole-[1,5-a]-pyrimidine-3,5-diamine, la 2,7-diméthylpyrazole-[1,5-a]-pyrimidine-3,5-diamine, le 3-aminopyrazole-[1,5-a]-pyrimidin-7-ol, le 3-aminopyrazole-[1,5-a]-pyrimidin-5-ol, le 2-(3-aminopyrazole-[1,5-a]-pyrimidin-7-ylamino)-éthanol, le 2-(7-aminopyrazole-[1,5-a]-pyrimidin-3-ylamino)-éthanol, le 2-[(3-aminopyrazole-[1,5-a]-pyrimidin-7-yl)-(2-hydroxyéthyl)-amino]-éthanol, le 2-[(7-aminopyrazole-[1,5-a]-pyrimidine-3-yl)-(2-hydroxyéthyl)-amino]-éthanol, la 5,6-diméthylpyrazole-[1,5-a]-pyrimidine-3,7-diamine, la 2,6-diméthylpyrazole-[1,5-a]-pyrimidine-3,7-diamine et la 3-amino-7-diméthylamino-2,5-diméthylpyrazole-[1,5-a]-pyrimidine ou un des sels physiologiquement acceptables de ces composés.

9. Agent selon l'une quelconque des revendications 1 à 8, **caractérisé en ce qu'**il contient au moins encore un autre composant de développement.

10. Agent selon la revendication 9, **caractérisé en ce que** l'autre composant de développement est choisi parmi la p-phénylènediamine, la p-toluylènediamine, le p-aminophénol, le 1-(2'-hydroxyéthyl)-2,5-diaminobenzène, le 1,10-bis-(2',5'-diaminophényl)-1,4,7,10-tétraoxydécane, la N,N-bis-(2'-hydroxyéthyl)-p-phénylènediamine, le 4-amino-3-méthylphénol, le 4-amino-2-((diéthylamino)-méthyl)-phénol, le 4-amino-2,6-dichlorophénol ou le 2-aminométhyl-4-aminophénol ainsi que les sels physiologiquement acceptables de ces composés.

11. Agent selon l'une quelconque des revendications 1 à 10, **caractérisé en ce qu'**il contient au moins un composant de couplage.

12. Agent selon la revendication 11, **caractérisé en ce que** le composant de couplage est choisi parmi le 1-naphtol, le 1,5-dihydroxynaphtalène, le 1,7-dihydroxynaphtalène, le 2,7-dihydroxynaphtalène, le 3-aminophénol, le 5-amino-2-méthylphénol, le 5-(2'-hydroxyéthylamino)-3-méthylphénol, le 2,4-dichloro-3-aminophénol, le 2-chloro-6-méthyl-3-aminophénol, le 2-méthyl-4-chloro-5-aminophénol, le résorcinol, l'ester monométhylique du résorcinol, le 2-chlororésorcinol, le 4-chlororésorcinol, le 2-méthylrésorcinol, le 5-méthylrésorcinol, le 2,5-diméthylrésorcinol, la m-phénylènediamine, la 2-méthylamino-3-amino-6-méthoxypyridine, le 2,4-diaminophénoxyéthanol, le 1,3-bis-(2',4'-diaminophénoxy)-propane, la 2-amino-3-hydroxypyridine ou le 2,6-dihydroxy-3,4-diméthylpyridine ainsi que parmi les sels physiologiquement acceptables de ces composés.

13. Agent selon l'une quelconque des revendications 1 à 12, **caractérisé en ce qu'**il contient en outre des précurseurs de colorants analogues aux colorants naturels, tels que les dérivés d'indole et d'indoline,

14. Agent selon l'une quelconque des revendications 1 à 13, **caractérisé en ce que** les composants de développement sont contenus en une quantité de 0,005 à 20% en poids, de préférence de 0,1 à 5% en poids et les composants de couplage en une quantité de 0,005 à 20% en poids, de préférence de 0,1 à 5% en poids, à chaque fois par rapport à la totalité de l'agent de teinture par oxydation.

15. Agent selon l'une quelconque des revendications 1 à 14, **caractérisé en ce qu'**il contient en outre au moins un colorant direct.

16. Procédé pour la teinture de fibres kératiniques, **caractérisé en ce qu'**un agent pour la teinture de fibres kératiniques selon l'une quelconque des revendications 1 à 15 est appliqué dans une préparation cosmétique appropriée sur les fibres à teinter et est à nouveau éliminé par rinçage ou lavage avec un shampooing après un temps d'action.

17. Utilisation d'un agent selon l'une quelconque des revendications 1 à 15 pour la teinture de fibres kératiniques.
